# EUROPEAN PATENT SPECIFICATION

(11) **EP 2 878 269 B1**
(45) Date of publication and mention of the grant of the patent: **21.12.2022**
(21) Application number: 14182591.9
(22) Date of filing: 28.08.2014
(51) Int. Cl.: A61B 8/00, B06B 1/06, H01L 41/08

(54) **Ultrasonic probe**
Ultraschallsonde
Sonde ultraacoustique

(30) Priority: 29.10.2013 KR 20130129098
(43) Date of publication of application: 03.06.2015
(73) Proprietor: Samsung Medison Co., Ltd., Gangwon-do 250-870 (KR)
(72) Inventor: Jin, Gil Ju, Seoul (KR); Kwon, Sung Do, Gyeonggi-do (KR)
(74) Representative: Frey, Sven Holger

(56) References cited:
- WO-A2-2013/140298
- JP-A- 2013 146 478
- US-A1- 2008 139 974
- US-A1- 2009 287 086

## Description

### BACKGROUND

### 1. Field

Embodiments of the present invention relate to an ultrasonic probe for generating an image of an inside of a subject using ultrasonic waves and an ultrasonic imaging apparatus having the same.

### 2. Description of the Related Art

Ultrasonic diagnosis apparatuses are apparatuses that radiate ultrasonic signals onto a target part of a body from a body surface of the subject and obtain a tomogram of a soft tissue or an image of a blood flow of the soft tissue using information regarding reflected ultrasonic signals (ultrasonic echo signals) in a noninvasive manner.

In comparison with other image diagnosis apparatuses, such as X-ray diagnosis apparatuses, X-ray computerized tomography (CT) scanners, magnetic resonance imaging (MRI) apparatuses, and nuclear medicine apparatuses, ultrasonic diagnosis apparatuses are small-sized and cheap, can be displayed in real time and have high safety due to lack of radiation exposure. Thus, ultrasonic diagnosis apparatuses are widely used in heart, abdominal, urinary, and ob-gyn diagnoses.

Meanwhile, an ultrasonic probe that radiates ultrasonic waves may include a transducer installed in the ultrasonic probe. An inner side of the ultrasonic probe is required to be maintained at an appropriate temperature so that the transducer operates normally.

Patent Documents WO 2013/140298 and US 2008/139974 also disclose heat dissipation strategies for ultrasonic probes.

### SUMMARY

Therefore, it is an aspect of the present invention to provide an ultrasonic probe that controls temperature of a piezoelectric layer using a thermoelectric element.

Additional aspects of the invention will be set forth in part in the description which follows and, in part, will be obvious from the description, or may be learned by practice of the invention.

In accordance with one aspect of the present invention, an ultrasonic probe according to claim 1 is disclosed.

In accordance with still another aspect of the disclosure, not part of the invention, an ultrasonic imaging apparatus includes: an ultrasonic probe including a piezoelectric layer that vibrates and generates ultrasonic waves, a thermoelectric element that contacts one surface of the piezoelectric layer and controls temperature of the piezoelectric layer according to a supplied current, and a temperature sensor that senses the temperature of the piezoelectric layer; and a controller that controls the supplied current based on the sensed temperature.

In accordance with yet still another aspect of the disclosure, not part of the invention, an ultrasonic imaging apparatus includes: an ultrasonic probe including a piezoelectric layer that vibrates and generates ultrasonic waves, an application specific integrated circuit (ASIC) that transfers a current to the piezoelectric layer, a thermoelectric element that contacts one surface of the ASIC and controls temperature of the piezoelectric layer according to the supplied current, and a temperature sensor that senses the temperature of the piezoelectric layer; and a controller that controls the supplied current based on the sensed temperature.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a perspective view of an ultrasonic imaging apparatus in accordance with an embodiment of the present invention;
FIG. 2A is a perspective of an ultrasonic probe according to the related art, and FIG. 2B is an exploded perspective view of the ultrasonic probe illustrated in FIG. 2A;
FIG. 3 illustrates an ultrasonic probe including a thermoelectric element, in accordance with an embodiment of the present invention;
FIGS. 4A through 4C illustrate ultrasonic probes including a signal electrode and a ground electrode, in accordance with other embodiments of the present invention;
FIGS. 5A through 5E illustrate ultrasonic probes including an application specific integrated circuit (ASIC), in accordance with various embodiments of the present invention;
FIGS. 6A and 6B illustrate ultrasonic probes in which a piezoelectric layer is diced, in accordance with other embodiments of the present invention; and
FIG. 7 is a control block diagram of an ultrasonic imaging apparatus including the ultrasonic probe in which the thermoelectric element is disposed, in accordance with an embodiment of the present invention.

### DETAILED DESCRIPTION

Reference will now be made in detail to the embodiments of the present invention, examples of which are illustrated in the accompanying drawings, wherein like reference numerals refer to like elements throughout.

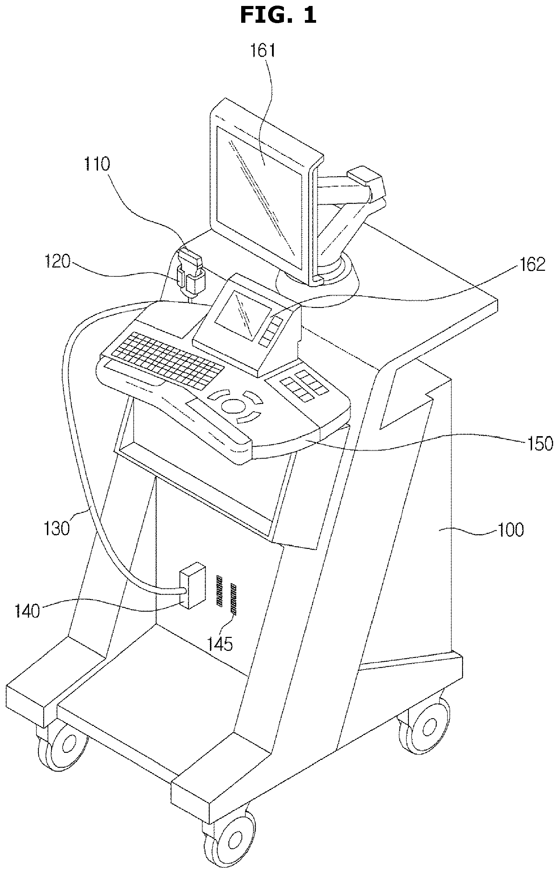

FIG. 1 is a perspective view of an ultrasonic imaging apparatus in accordance with an embodiment of the present disclosure.

As illustrated in FIG. 1, the ultrasonic imaging apparatus may include a body 100, an ultrasonic probe 110, an input unit 150, and a display 160. In this case, the display 160 may include a main display 161 and a subdisplay 162.

One or more female connectors 145 may be provided at one side of the body 100. A male connector 140 connected to a cable 130 may be physically coupled to the female connector 145.

A plurality of casters (not shown) for mobility of the ultrasonic imaging apparatus may be provided at a lower portion of the body 100. The plurality of casters (not shown) may fix the ultrasonic imaging apparatus at a particular place or may move the ultrasonic imaging apparatus in a particular direction.

The ultrasonic probe 110 that contacts a body surface of a subject may transmit and receive ultrasonic waves. One end of the cable 130 may be connected to the ultrasonic probe 110, and the male connector 140 may be connected to the other end of the cable 130. The male connector 140 connected to the other end of the cable 130 may be physically coupled to the female connector 145 of the body 100.

Instructions relating to an operation of the ultrasonic imaging apparatus may be input to the input unit 150. For example, mode selection instructions, such as an amplitude mode (A-mode), a brightness mode (B-mode), and a motion mode (M-mode), or ultrasonic diagnosis starting instructions may be input to the input unit 150. The instructions input through the input unit 150 may be transmitted to the body 100 through wired or wireless communication.

The input unit 150 may include at least one of a keyboard, a foot switch, and a foot pedal, for example. The keyboard may be implemented with hardware and may be placed at an upper portion of the body 100. The keyboard may include at least one of a switch, a key, a joystick, and a trackball. Alternatively, the keyboard may be implemented with software, like a graphic user interface. In this case, the keyboard may be displayed on the subdisplay 162 or the main display 161. The foot switch or the foot pedal may be disposed at the lower portion of the body 100, and a manipulator may control the operation of the ultrasonic imaging apparatus using the foot pedal.

An ultrasonic probe holder 120 for holding the ultrasonic probe 110 may be disposed around the input unit 150. One or more ultrasonic probe holders 120 may be provided. When an inspector does not use the ultrasonic imaging apparatus, the ultrasonic probe 110 may be held by and kept in the ultrasonic probe holder 120.

The subdisplay 162 may be disposed on the body 100. FIG. 1 illustrates a case that the subdisplay 162 is disposed at an upper portion of the input unit 150. The subdisplay 162 may display an application relating to the operation of the ultrasonic imaging apparatus. For example, the subdisplay 162 may display a menu or information required for ultrasonic diagnosis. The subdisplay 162 may be implemented with a cathode ray tube (CRT) or a liquid crystal display (LCD), for example.

The main display 161 may be disposed at the body 100. FIG. 1 illustrates a case that the main display 161 is disposed at an upper portion of the subdisplay 162. The main display 161 may display an ultrasonic image obtained during an ultrasonic diagnosis procedure. The main display 161 may be implemented with a CRT or an LCD, like in the subdisplay 162. Although FIG. 1 illustrates a case that the main display 161 is coupled to the body 100, the main display 161 may be implemented to be detachable from the body 100.

FIG. 1 illustrates a case that both the main display 161 and the subdisplay 162 are provided at the ultrasonic imaging apparatus. However, the subdisplay 162 may be omitted, as needed. In this case, the application or menu displayed on the subdisplay 162 may be displayed on the main display 161.

Until now, a general configuration of the ultrasonic imaging apparatus has been described. Hereinafter, an ultrasonic probe according to the present invention will be described in more detail.

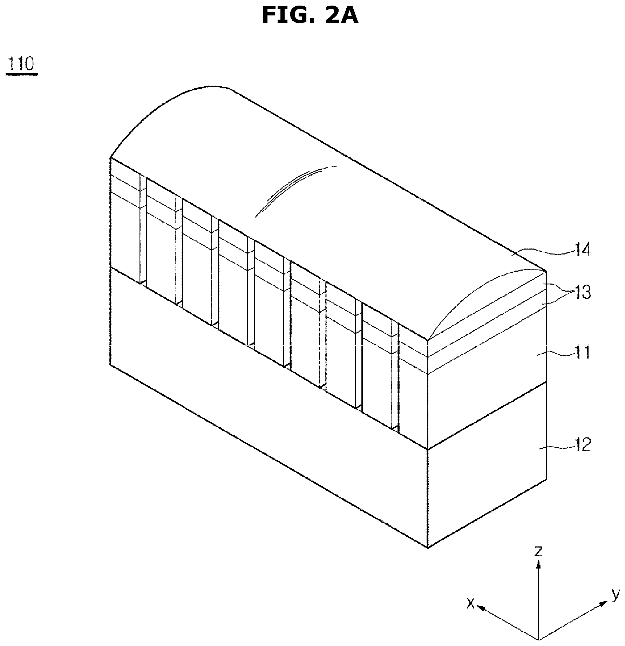

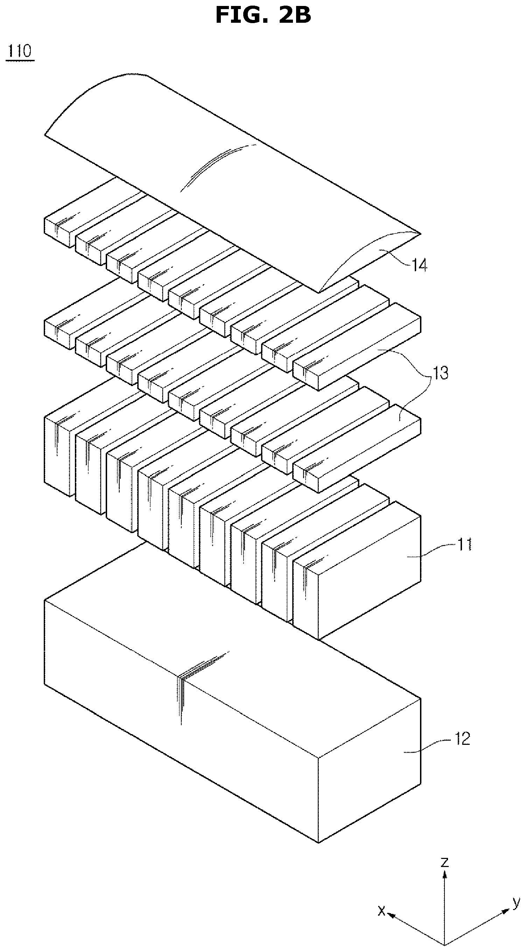

FIG. 2A is a perspective of an ultrasonic probe according to the related art, and FIG. 2B is an exploded perspective view of the ultrasonic probe illustrated in FIG. 2A.

An ultrasonic probe 110 according to the related art may include a piezoelectric layer 11, a matching layer 13 disposed on a top surface of the piezoelectric layer 11, a lens 14 installed at a top surface of the matching layer 13, and a backing layer 12 disposed on a bottom surface of the piezoelectric layer 11.

The effects that, if mechanical pressure is applied to a predetermined material, a voltage is generated and if a voltage is applied to the predetermined material, mechanical deformation occurs, are referred to as a piezoelectric effect and an inverse piezoelectric effect, and a material having these effects is referred to as a piezoelectric material. That is, the piezoelectric material is a material that transforms electric energy into mechanical vibration energy or vice versa.

The ultrasonic probe 110 of FIG. 1 includes the piezoelectric layer 11 that transforms a received electrical signal into mechanical vibration so as to generate ultrasonic waves.

The piezoelectric material that constitutes the piezoelectric layer 11 may include a ceramic of lead zirconate titanate (PZT), a PZMT monocrystal formed of a solid solution of lead magnesium niobate (PMN) and lead titanate (PT), or a PZNT monocrystal formed of a solid solution of lead zinc niobate (PZN) and PT.

The backing layer 12 is mounted on the bottom surface of the piezoelectric layer 11, absorbs the ultrasonic waves generated in the piezoelectric layer 11, and blocks the ultrasonic waves that proceed toward the bottom surface of the piezoelectric layer 11, thereby preventing distortion of an image. The backing layer 12 may be manufactured as a plurality of layers so as to improve an attenuation or blocking effect of the ultrasonic waves.

The matching layer 13 is disposed on the top surface of the piezoelectric layer 11 and reduces a difference in sound impedance between the piezoelectric layer 11 and the subject so that ultrasonic waves generated in the piezoelectric layer 11 can be effectively transferred to the subject. The matching layer 13 may be formed to have one or more layers.

The matching layer 13 may be divided into a plurality of units having predetermined widths together with the piezoelectric layer 11 through a dicing process.

Although not shown, a protective layer may be disposed on the top surface of the matching layer 13. The protective layer may prevent outflow of high frequency components that may be generated in the piezoelectric layer 11 and may block inflow of external high frequency signals. Also, the protective layer may protect internal parts of the ultrasonic probe from water or medicine used in disinfection by coating or depositing a conductive material on the surface of a film having moisture tolerance and chemical resistance.

The lens 14 is installed at the top surface of the matching layer 13. The lens 14 may have a convex shape in a radiation direction of the ultrasonic waves so as to focus the ultrasonic waves, and when sound speed is slower than the human body, the lens 14 may be implemented with a concave shape.

Meanwhile, all parts of vibration of the piezoelectric layer 11 are not transformed into ultrasonic waves but parts thereof are converted into heat, and thus a sound loss occurs. Heat generated in this way may increase an internal temperature of the ultrasonic probe 110 to 40°C or higher. Since the high-temperature ultrasonic probe 110 directly contacts a skin, a patient's health may be under threat. If heat generated in the piezoelectric layer 11 is not properly dissipated, performance of the ultrasonic probe 110 may be adversely affected. In particular, a depolarization phenomenon may occur in the piezoelectric material that constitutes the piezoelectric layer 11 in a high-temperature environment. This may cause performance of the piezoelectric layer 11 to be degraded.

When the ultrasonic probe 110 is in a high-temperature state and in an extremely low temperature environment, performance degradation may occur. Thus, the temperature of the ultrasonic probe 110, in detail, the temperature of the piezoelectric layer 11 is required to be uniformly maintained regardless of a peripheral environment.

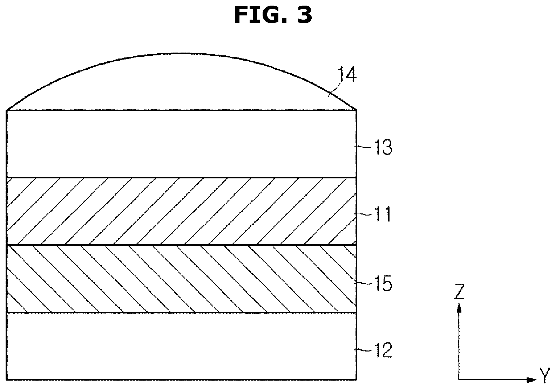

FIG. 3 illustrates an ultrasonic probe including a thermoelectric element, in accordance with an embodiment of the present disclosure.

As illustrated in FIG. 3, a thermoelectric element 15 may contact one surface of the piezoelectric layer 11 so as to control the temperature of the piezoelectric layer 11. Here, the thermoelectric element 15 is an element that uses the peltier effect that one terminal causes a heat absorption reaction and the other terminal causes a heat dissipation reaction according to a direction of a current if ends of different types of metals are connected to each other and a current flows through the connected metal ends.

In an embodiment of the thermoelectric element 15, the thermoelectric element 15 may include a heat dissipation plate that is disposed at one surface of the thermoelectric element 15 and dissipates heat, and a heat absorption plate that is disposed at an opposite side to the heat dissipation plate and absorbs heat.

In this case, the used thermoelectric element 15 may be a thin film type thermoelectric element 15. The thickness of the thermoelectric element 15 may be several tens of micrometers (um) to several hundreds of micrometers (um). The thinner the thermoelectric element 15, the less effect on sound performance of the piezoelectric layer 11, but performance of the thermoelectric element 15 is degraded. Thus, an appropriate thickness of the thermoelectric element 15 may be determined according to situations and may be applied to the ultrasonic probe 110.

If the thermoelectric element 15 contacts one surface of the piezoelectric layer 11 through the heat dissipation plate, the heat dissipation plate may dissipate heat and may heat the piezoelectric layer 11. In contrast, if the thermoelectric element 15 contacts one surface of the piezoelectric layer 11 through the heat absorption plate, the heat absorption plate may absorb heat generated in the piezoelectric layer 11 and may cool the piezoelectric layer 11.

FIG. 3 illustrates the ultrasonic probe 110 in which the thermoelectric element 15 contacts a bottom surface of the piezoelectric layer 11, in accordance with an embodiment of the present disclosure.

The piezoelectric layer 11 and the thermoelectric element 15 may make surface contact with each other and may be configured such that conduction of heat may be easily performed. In this case, a thermal compound may be used to improve the effects of surface contact.

When ultrasonic diagnosis is performed in an extremely low-temperature environment, the piezoelectric layer 11 needs to be heated for a normal operation of the ultrasonic probe 110. To this end, the heat dissipation plate may be disposed on a top surface of the thermoelectric element 15, i.e., on a surface that makes surface contact with the piezoelectric layer 11. Heat generated in the heat dissipation plate provided in this way may be transferred to the piezoelectric layer 11, and the piezoelectric layer 11 to which heat is transferred, may be maintained at a uniform temperature and may generate ultrasonic waves normally.

Alternatively, an internal temperature of the ultrasonic probe 110 may be increased due to heat generated due to vibration of the piezoelectric layer 11. If heat generated in this way is not dissipated, normal ultrasonic diagnosis becomes difficult. To this end, the heat absorption plate may be disposed on the top surface of the thermoelectric element 15, i.e., on a surface that makes surface contact with the piezoelectric layer 11. Heat generated in the piezoelectric layer 11 is absorbed by the heat absorption plate so that the piezoelectric layer 11 may be cooled and the internal temperature of the ultrasonic probe 110 may be prevented from being increased.

As a result, the piezoelectric layer 11 and the thermoelectric element 15 make surface contact with each other so that the piezoelectric layer 11 can be maintained at a uniform temperature and the ultrasonic probe 110 can operate normally.

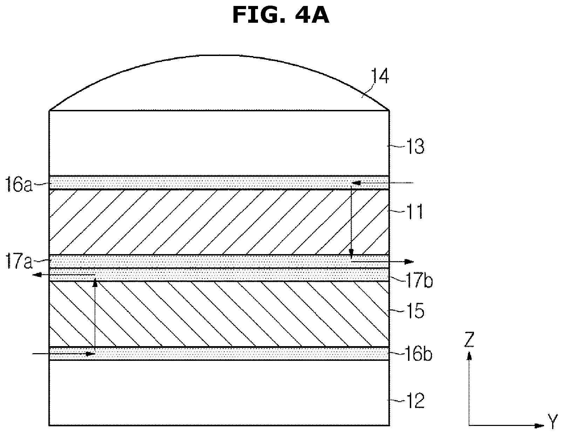

FIGS. 4A through 4C illustrate ultrasonic probes including a signal electrode and a ground electrode, in accordance with embodiments of the present disclosure. Arrows in FIGS. 4A through 4C mean a direction in which a current flows.

The current may be supplied to the piezoelectric layer 11 so that the piezoelectric layer 11 generates ultrasonic waves. To this end, the piezoelectric layer 11 may include a signal electrode 16a for the piezoelectric layer 11 to which the current is supplied, and a ground electrode 17a for the piezoelectric layer 11 through which the current is discharged. If the current is supplied to the piezoelectric layer 11 via the signal electrode 16a for the piezoelectric layer 11, the piezoelectric layer 11 vibrates and generates ultrasonic waves.

Since the thermoelectric element 15 generates a temperature difference between both ends of the thermoelectric element 15 due to the peltier effect, a supply of current is required. To this end, the thermoelectric element 15 may include a signal electrode 16b for the thermoelectric element 15 to which a current is supplied, and a ground electrode 17b for the thermoelectric element 15 through which the current is discharged. The thermoelectric element 15 generates a temperature difference between both ends of the thermoelectric element 15 due to the current supplied to the thermoelectric element 15 through the signal electrode 16b, thereby controlling the temperature of the piezoelectric layer 11.

FIG. 4A illustrates the ultrasonic probe 110 in which the piezoelectric layer 11 and the thermoelectric element 15 make surface contact with each other, i.e., a case that a signal electrode and a ground electrode are disposed on the piezoelectric layer 11 an the thermoelectric element 15, respectively, in accordance with an embodiment of the present disclosure.

In this case, a current is supplied to the piezoelectric layer 11 through the signal electrode 16a for the piezoelectric layer 11, and a current is supplied to the thermoelectric element 15 through the signal electrode 16b for the thermoelectric element 15. Thus, the piezoelectric layer 11 and the thermoelectric element 15 operate due to separate currents.

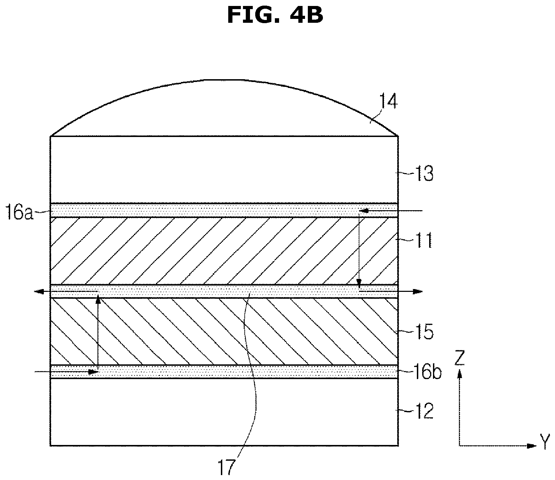

FIG. 4B illustrates the ultrasonic probe 110 in which the piezoelectric layer 11 and the thermoelectric element 15 make surface contact with each other, i.e., a case that the piezoelectric layer 11 and the thermoelectric element 15 share a ground electrode, in accordance with another embodiment of the present disclosure.
In this case, a current is supplied to the piezoelectric layer 11 through the signal electrode 16a for the piezoelectric layer 11, and a current is supplied to the thermoelectric element 15 through the signal electrode 16b for the thermoelectric element 15, like in FIG. 4A. However, there is a difference between FIGS. 4A and 4B in that, in FIG. 4B, the current flows to the ground through one ground electrode. Hereinafter, the ground electrode shared by the piezoelectric layer 11 and the thermoelectric element 15 is referred to as a shared ground electrode 17.

As illustrated in FIG. 4B, if the piezoelectric layer 11 and the thermoelectric element 15 share the ground electrode, only one ground electrode is sufficient. Connection lines connected to the outside are required to be provided in each electrode. If the number of electrodes is reduced, the number of connection lines that accompany with the number of electrodes is also reduced to overcome a limitation in an internal space of the ultrasonic probe 110.

Also, even when the piezoelectric layer 11 and the thermoelectric element 15 are diced, the risk of generating an electrical short is low. In addition, interference that occurs between a plurality of electrode layers is reduced so that sound performance can be uniformly maintained.

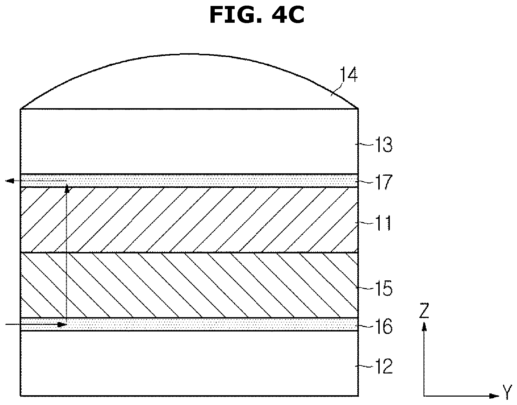

FIG. 4C illustrates the ultrasonic probe 110 in which the piezoelectric layer 11 and the thermoelectric element 15 make surface contact with each other, i.e., a case that the piezoelectric layer 11 and the thermoelectric element 15 share a ground electrode and a signal electrode, in accordance with still another embodiment of the present disclosure.

Hereinafter, the signal electrode shared by the piezoelectric layer 11 and the thermoelectric element 15 is referred to as a shared signal electrode 16.

Referring to FIG. 4C, a current may be supplied to the thermoelectric element 15 via the shared signal electrode 16 disposed on a bottom surface of the thermoelectric element 15. A surface of the thermoelectric element 15 on which the shared signal electrode 16 is disposed, may be a heat dissipation plate. When the supplied current flows from the heat dissipation plate to the heat absorption plate, a temperature difference may be generated in both ends of the thermoelectric element 15. As a result, heat generated in the piezoelectric layer 11 is absorbed by the heat absorption plate, and thus the piezoelectric layer 11 may be cooled.

Also, the current that flows through the heat absorption plate may be transferred to the piezoelectric layer 11 and may vibrate the piezoelectric layer 11. The current may be discharged through the shared ground electrode 17 disposed on a top surface of the piezoelectric layer 11.

As illustrated in FIG. 4C, if a current is supplied through the shared signal electrode 16, a temperature difference of the thermoelectric element 15 may be generated due to the supplied current, and the piezoelectric layer 11 may be vibrated, and ultrasonic waves may be generated. Unlike in FIG. 4A or 4B in which the piezoelectric layer 11 and the thermoelectric element 15 are controlled due to separate currents, the piezoelectric layer 11 and the thermoelectric element 15 are controlled when one current flows through the piezoelectric layer 11 and the thermoelectric element 15 via the shared signal electrode 16.

In this way, if the piezoelectric layer 11 and the thermoelectric element 15 share the signal electrode as well as the ground electrode, the number of electrodes and the number of connection lines that accompany with the number of electrodes are reduced such that there is no problem of the limitation in the internal space of the ultrasonic probe 110.

Also, even when the piezoelectric layer 11 and the thermoelectric element 15 are diced, there is a low possibility that an electrical short will occur. In addition, interference that occurs between a plurality of electrode layers is reduced so that sound performance is not degraded.

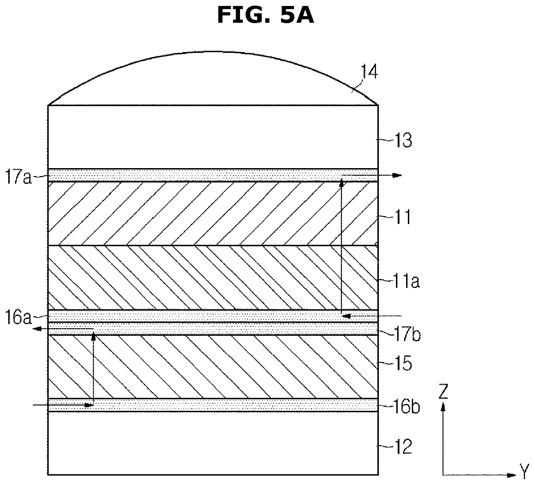

FIGS. 5A through 5E illustrate ultrasonic probes including an application specific integrated circuit (ASIC), in accordance with various embodiments of the present disclosure.

As the configuration of the ultrasonic probe 110 is recently diversified, complexity of an inside of the ultrasonic probe 110 increases. In particular, as an arrangement shape of a transducer device is complicated, the ultrasonic probe 110 may include an application specific integrated circuit (ASIC) so as to control a current supplied to each device. Owing to integration technology using the ASIC, the reliability of the ultrasonic probe 110 and the ultrasonic imaging apparatus can be increased, and complexity of the ultrasonic probe 110 can be decreased, and excellent signal processing efficiency can be obtained.

In this way, even when the ultrasonic probe 110 includes the ASIC so as to transfer the current to the piezoelectric layer 11, the ultrasonic probe 110 may include the thermoelectric element 15 so as to control the temperature of the piezoelectric layer 11.

FIGS. 5A through 5C illustrate the ultrasonic probe 110 in which an ASIC 11a makes surface contact with the bottom surface of the piezoelectric layer 11 and the thermoelectric element 15 makes surface contact with a bottom surface of the ASIC 11a, in accordance with various embodiments of the present disclosure. In this case, since the ASIC 11a controls the supplied current and transfers the current to the piezoelectric layer 11, each embodiment of the ultrasonic probe 110 including the ASIC 11a is subject to a case that the supplied current is transferred to the piezoelectric layer 11 via the ASIC 11a. Arrows of FIGS. 5A through 5E represent a direction in which the current flows.

FIG. 5A illustrates a case that the piezoelectric layer 11 includes a signal electrode and a ground electrode for the piezoelectric layer 11 and the thermoelectric element 15 includes a signal electrode and a ground electrode for the thermoelectric element 15. The signal electrode 16a for the piezoelectric layer 11 may be disposed on the ASIC 11a, and a current may be supplied to the signal electrode 16a for the piezoelectric layer 11. The current supplied in this way may be controlled by the ASIC 11a and may be transferred to the piezoelectric layer 11, and the piezoelectric layer 11 to which the current is transferred, may vibrate and generate ultrasonic waves. Also, the current is discharged along the ground electrode 17a disposed on the piezoelectric layer 11.

Meanwhile, the thermoelectric element 15 may include the signal electrode 16b and the ground electrode 17b for the thermoelectric element 15. In FIG. 5A, the signal electrode 16b for the thermoelectric element 15 is disposed on the bottom surface of the thermoelectric element 15. Thus, the bottom surface of the thermoelectric element 15 is a heat dissipation plate. The current supplied along the signal electrode 16b for the thermoelectric element 15 flows from the heat dissipation plate to an opposite site, i.e., to a heat absorption plate and is discharged along the ground electrode 17b for the thermoelectric element 15. In this case, the heat absorption plate absorbs heat generated in the piezoelectric layer 11 via the ASIC 11a and thus may cool the piezoelectric layer 11.

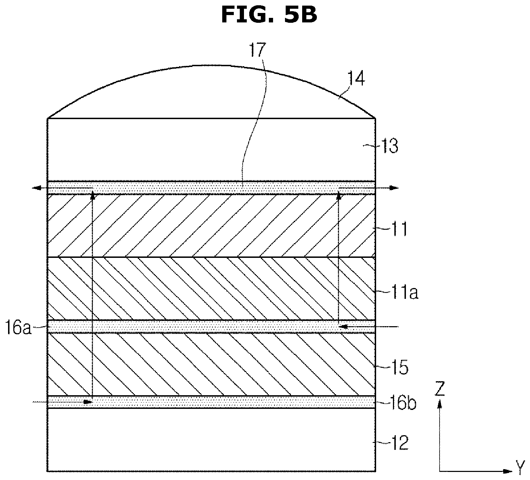

Unlike in FIG. 5A, FIG. 5B illustrates a case that the shared ground electrode 17 is disposed. The current supplied along the signal electrode 16a for the piezoelectric layer 11 disposed on the ASIC 11a vibrates the piezoelectric layer 11 and is discharged along the shared ground electrode 17. Also, the current supplied along the signal electrode 16b for the thermoelectric element 15 disposed on the thermoelectric element 15 generates a temperature difference in both ends of the thermoelectric element 15 and is discharged along the shared ground electrode 17 disposed on the thermoelectric element 15 via the ASIC 11a.

When the shared ground electrode 17 is disposed in this way, the number of ground electrodes may be reduced to one, and the number of connection lines that accompany with the number of ground electrodes may be reduced, too. Thus, the limitation in the internal space of the ultrasonic probe 110 can be overcome.

Also, even when the piezoelectric layer 11 and the thermoelectric element 15 are diced, the risk of generating an electrical short is low. In addition, interference that occurs between a plurality of electrode layers is reduced so that uniform sound performance can be maintained.

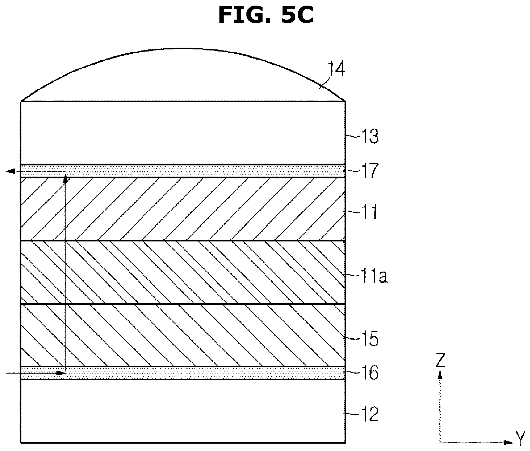

FIG. 5C illustrates a case that the shared signal electrode 16 as well as the shared ground electrode 17 are provided, unlike in FIG. 5B. Since the shared signal electrode 16 is disposed on the bottom surface of the thermoelectric element 15, the bottom surface of the thermoelectric element 15 may be a heat dissipation plate, and a top surface of the thermoelectric element 15 may be a heat absorption plate.

If a current is applied along the shared signal electrode 16, a current flows from the heat dissipation plate to the heat absorption plate and generates a temperature difference in both ends of the thermoelectric element 15. Also, the current is transferred from the thermoelectric element 15 to the ASIC 11a, is transferred again to the piezoelectric layer 11, and vibrates the piezoelectric layer 11. The current that is transmitted to the piezoelectric layer 11 is discharged along the shared ground electrode 17 disposed on the piezoelectric layer 11.

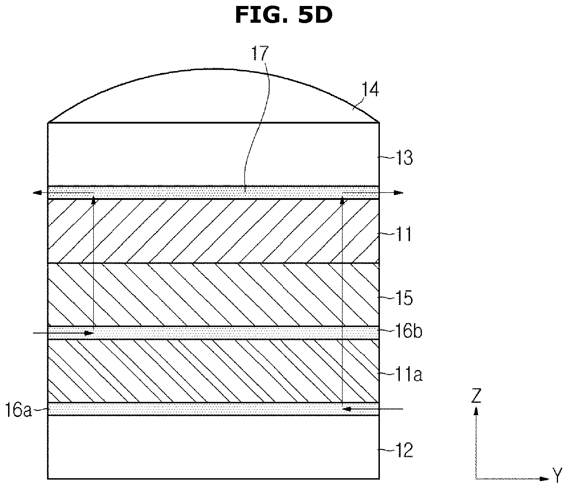

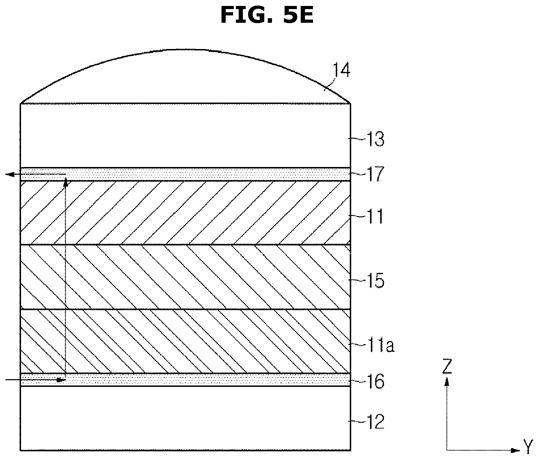

Unlike in FIGS. 5A through 5C, FIGS. 5D and 5E illustrate various embodiments of the ultrasonic probe 110 in which the thermoelectric element 15 makes surface contact with the bottom surface of the piezoelectric layer 11 and the ASIC 11a makes surface contact with the bottom surface of the thermoelectric element 15. Since the current controlled by the ASIC 11a may be transferred to the piezoelectric layer 11 via the thermoelectric element 15, this embodiment may be implemented.

FIG. 5D illustrates the ultrasonic probe 110 including the shared ground electrode, in accordance with another embodiment of the present disclosure. The signal electrode 16a for the piezoelectric layer 11 may be disposed on the ASIC 11a, and a current may be supplied via the signal electrode 16a for the piezoelectric layer 11. The supplied current may be transferred to the piezoelectric layer 11 via the thermoelectric element 15, and the piezoelectric layer 11 may vibrate due to the transferred current and may generate ultrasonic waves. The current that vibrates the piezoelectric layer 11 may be discharged through the shared ground electrode 17 disposed on the piezoelectric layer 11.

Also, the signal electrode 16b for the thermoelectric element 15 may be disposed on the bottom surface of the thermoelectric element 15. Thus, the bottom surface of the thermoelectric element 15 may be a heat dissipation plate that heats a contact surface between the piezoelectric layer 11 and the thermoelectric element 15, and the top surface of the thermoelectric element 15 may be a heat absorption plate that cools the contact surface between the piezoelectric layer 11 an the thermoelectric element 15. The current supplied through the signal electrode 16b for the thermoelectric element 15 may flow from the heat dissipation plate to the heat absorption plate and may generate a temperature difference in both ends of the thermoelectric element 15. The current that flows through the heat absorption plate may flow along the piezoelectric layer 11 and may be discharged along the shared ground electrode 17.

FIG. 5D is the same as FIG. 5B in that the number of ground electrodes is reduced to one and the weight or volume of the ultrasonic probe 110 may be reduced, unlike in FIG. 5A.

FIG. 5E illustrates the ultrasonic probe 110 including the shared ground electrode and the shared signal electrode, in accordance with still another embodiment of the present disclosure.

The shared signal electrode 16 may be disposed on the ASIC 11a, and a current that controls the thermoelectric element 15 and the piezoelectric layer 11 may be supplied through the shared signal electrode 16. Also, the shared ground electrode 17 may be disposed on the piezoelectric layer 11, and the supplied current may be discharged through the shared ground electrode 17.

Like in FIG. 5C, the number of electrodes is reduced to two, and there is no problem of the limitation in the internal space of the ultrasonic probe 110. Also, even when the piezoelectric layer 11 and the thermoelectric element 15 are diced, the risk of generating an electrical short is low. In addition, interference that occurs between a plurality of electrode layers is reduced so that sound performance can be maintained.

The ultrasonic probe 110 illustrated in FIGS. 2A and 2B, FIG. 3, FIGS. 4A through 4C, and FIGS. 5A through 5E is a one-dimensional arrangement ultrasonic probe 110. However, even when the transducer device is arranged in a two-dimensional manner, the temperature of the piezoelectric layer 11 may be controlled through the thermoelectric element 15.

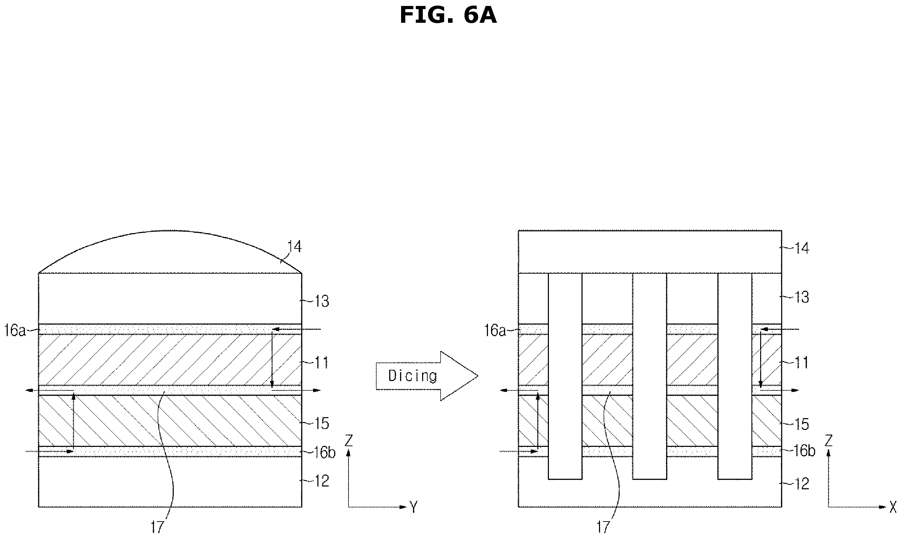

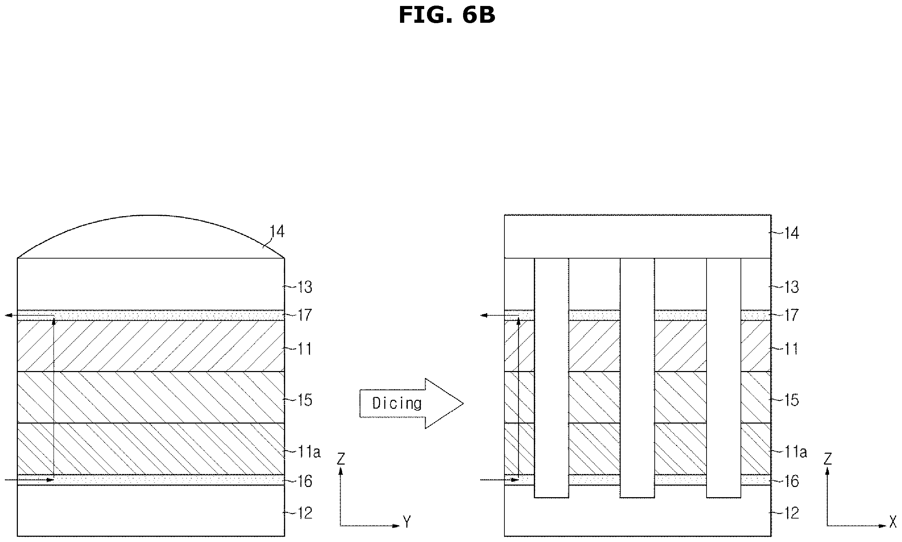

FIGS. 6A and 6B illustrate ultrasonic probes in which the piezoelectric layer 11 is diced, in accordance with other embodiments of the present disclosure. Arrows in FIGS. 6A and 6B mean a direction in which a current flows.

Even when the piezoelectric layer 11 is diced into a plurality of channels, temperature of the piezoelectric layer 11 may be controlled using the thermoelectric element 15. In detail, the piezoelectric layer 11 may be diced into and may include one or more piezoelectric channels. In this case, the piezoelectric layer 11 needs to be maintained at an appropriate temperature at which each piezoelectric channel operates normally. To this end, the thermoelectric element 15 including one or more thermoelectric channels may contact the piezoelectric layer 11. In particular, the thermoelectric element 15 may be diced into one or more thermoelectric channels, and each piezoelectric channel may contact each thermoelectric channel. Thus, temperature of each piezoelectric channel may be controlled by each thermoelectric channel.

FIG. 6A illustrates the ultrasonic probe 110 in which the thermoelectric element 15 makes surface contact with the piezoelectric layer 11 diced into one or more channels, in accordance with an embodiment of the present disclosure. Like in FIG. 6A, since the piezoelectric layer 11 includes one or more piezoelectric channels, the thermoelectric element 15 needs to be provided to contact each piezoelectric channel. To this end, the thermoelectric element 15 is diced into one or more thermoelectric channels and is provided to make surface contact with the piezoelectric layer 11.

FIG. 6B illustrates the ultrasonic probe 110 in which the ASIC 11a makes surface contact with the piezoelectric layer 11 diced into one or more channels and the diced thermoelectric element 15 makes surface contact with the ASIC 11a, in accordance with another embodiment of the present invention. Even when the ASIC 11a makes surface contact with the piezoelectric layer 11, the thermoelectric element 15 diced in the same manner as FIG. 6A may control temperature of the piezoelectric layer 11. If each thermoelectric channel makes surface contact with the ASIC 11a so as to maintain temperature of each piezoelectric channel that makes surface contact with the ASIC 11a, heat exchange between the piezoelectric channel and the thermoelectric channel may be performed by the ASIC 11a.

When the piezoelectric layer 11 is diced into one or more piezoelectric channels, the thermoelectric element 15 may be a thin film type thermoelectric element 15 so that the thermoelectric element 15 can be diced into one or more thermoelectric channels.

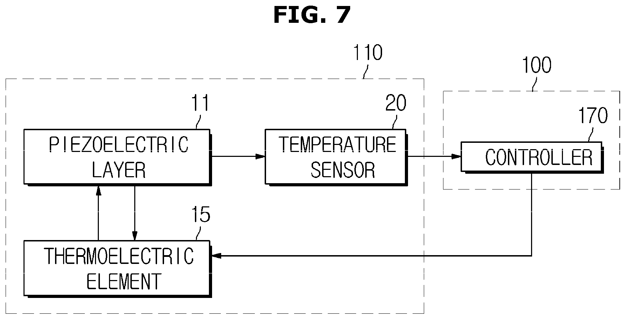

FIG. 7 is a control block diagram of an ultrasonic imaging apparatus including an ultrasonic probe in which a thermoelectric element is disposed, in accordance with an embodiment of the present disclosure.

The ultrasonic probe 110 may include the piezoelectric layer 11 that vibrates and generates ultrasonic waves, the thermoelectric element 15 that controls temperature of the piezoelectric layer 11, and a temperature sensor 20 that senses the temperature of the piezoelectric layer 11.

When the temperature of the piezoelectric layer 11 rises due to its vibration or decreases in an extremely low temperature environment, the piezoelectric layer may not operate normally. Thus, the temperature of the piezoelectric layer 11 needs to be maintained at an appropriate level.

To this end, the thermoelectric element 15 may contact one surface of the piezoelectric layer 11 or may make surface contact with the piezoelectric layer 11 and may contract one surface of the ASIC 11a that transfers a current. If a current is supplied to the thermoelectric element 15, a temperature difference is generated in both ends of the thermoelectric element 15, and thus, temperature of the piezoelectric layer 11 may be controlled using the temperature difference.

In detail, the thermoelectric element 15 may include a heat dissipation plate that dissipates heat toward a contact surface between the piezoelectric layer 11 and the thermoelectric element 15 and heats the contact surface, and a heat absorption plate that absorbs heat of the contact surface and cools the contact surface. The heat dissipation plate or the heat absorption plate contacts the piezoelectric layer 11 in consideration of an environment in which ultrasonic diagnosis is performed, so that the piezoelectric layer 11 may be heated or cooled.

The temperature sensor 20 may be disposed at an inner side of a housing and may sense the temperature of the piezoelectric layer 11. To this end, the temperature sensor 20 may be disposed to contact the piezoelectric layer 11. If it is determined that the temperature sensor 20 senses the temperature of the piezoelectric layer 11 and determines that the piezoelectric layer 11 is at a predetermined temperature or more or less, the result of determination may be transmitted to a controller 170 provided on the body 100.

The controller 170 may receive the result of sensing temperature from the temperature sensor 20 and may control a supply current supplied to the thermoelectric element 15 based on the result of sensing temperature. Since it is determined that temperature of the piezoelectric layer 11 is at an appropriate level or more or less, the controller 170 may control the ultrasonic imaging apparatus to supply a current to the thermoelectric element 15 and to cool or heat the piezoelectric layer 11. The controller 170 may include a processor and may be implemented with hardware.

In the ultrasonic probe 110 and the ultrasonic imaging apparatus including the same according to the one or more embodiments of the present disclosure, the piezoelectric layer 11 disposed in the ultrasonic probe 110 and the thermoelectric element 15 may make surface contact with each other directly or through the ASIC 11a so that the temperature of the piezoelectric layer 11 can be maintained in an optimum state. Accordingly, smooth ultrasonic diagnosis can be performed, and a patient's safety can be maintained.

As described above, in an ultrasonic probe and an ultrasonic imaging apparatus having the same according to the one or more embodiments of the present disclosure, a piezoelectric layer is heated or cooled using a thermoelectric element so that an inner side of the ultrasonic probe can be always maintained at an appropriate temperature.

In addition, an internal structure of the ultrasonic probe can be simplified by integrating electrodes of the thermoelectric element with electrodes of the piezoelectric layer.

## Claims

1. An ultrasonic probe (110) comprising:
a piezoelectric layer (11) that vibrates and generates ultrasonic waves if a current is supplied to the piezoelectric layer (11);
a thermoelectric element (15) that contacts one surface of an application specific integrated circuit (ASIC, 11a) and
controls the temperature of the piezoelectric layer (11) if a current is supplied to the thermoelectric element (15), with the bottom surface of the thermoelectric element (15) being a heat dissipation plate and the top surface of the thermoelectric element (15) being a heat absorption plate, and
the ASIC (11a) being disposed between the piezoelectric layer (11) and the thermoelectric element (15), wherein the ASIC (11a) makes surface contact with the bottom surface of the piezoelectric layer (11) and is configured to control a current transmitted to the piezoelectric layer (11);
a shared signal electrode (16) disposed on the bottom surface of the thermoelectric element (15) and configured to supply current;
a shared ground electrode (17) disposed on the upper surface of the piezoelectric layer (11) and configured to discharge current flowing through the ASIC (11a), the thermoelectric element (15) and the piezoelectric layer (11).

2. The ultrasonic probe (110) of claim 1, wherein the piezoelectric layer (11) and the thermoelectric element (15) share a signal electrode (16a,16b) through which a current is supplied to the piezoelectric layer (11) and the thermoelectric element (15), respectively.

3. The ultrasonic probe (110) of claim 1, wherein the piezoelectric layer (11) and the thermoelectric element (15) share a ground electrode (17a,17b).

4. The ultrasonic probe (110) of claim 1, wherein, when the piezoelectric layer (11) comprises one or more piezoelectric channels, the thermoelectric element (15) comprises one or more thermoelectric channels corresponding to the one or more piezoelectric channels.

## Patentansprüche

1. Ultraschallsonde (110), die Folgendes aufweist:
eine piezoelektrische Schicht (11), die vibriert und Ultraschallwellen erzeugt, wenn ein Strom an die piezoelektrische Schicht (11) angelegt wird;
ein thermoelektrisches Element (15), das eine Fläche einer anwendungsspezifischen integrierten Schaltung, ASIC (11a) kontaktiert und die Temperatur der piezoelektrischen Schicht (11) steuert, wenn ein Strom an das thermoelektrische Element (15) angelegt wird, wobei die untere Fläche des thermoelektrischen Elements (15) eine Wärmeableitungsplatte ist und die obere Fläche des thermoelektrischen Elements (15) eine Wärmeabsorptionsplatte ist, und
die ASIC (11a) zwischen der piezoelektrischen Schicht (11) und dem thermoelektrischen Element (15) angeordnet ist, wobei die ASIC (11a) flächigen Kontakt mit der unteren Fläche der piezoelektrischen Schicht (11) herstellt und dafür vorgesehen ist, einen an die piezoelektrische Schicht (11) angelegten Strom zu steuern;
eine gemeinsame Signalelektrode (16), die auf der unteren Fläche des thermoelektrischen Elements (15) angeordnet und dafür vorgesehen ist, Strom zu liefern;
eine gemeinsame Masseelektrode (17), die auf der oberen Fläche der piezoelektrischen Schicht (11) angeordnet und dafür vorgesehen ist, den durch die ASIC (11a), das thermoelektrische Element (15) und die piezoelektrische Schicht (11) fließenden Strom abzuführen.

2. Ultraschallsonde (110) nach Anspruch 1, wobei sich die piezoelektrische Schicht (11) und das thermoelektrische Element (15) eine Signalelektrode (16a, 16b) teilen, über die ein Strom an die piezoelektrische Schicht (11) und das thermoelektrische Element (15) zugeführt wird.

3. Ultraschallsonde (110) nach Anspruch 1, wobei sich die piezoelektrische Schicht (11) und das thermoelektrische Element (15) eine Masseelektrode (17a, 17b) teilen.

4. Ultraschallsonde (110) nach Anspruch 1, wobei, wenn die piezoelektrische Schicht (11) einen oder mehrere piezoelektrische Kanäle aufweist, das thermoelektrische Element (15) einen oder mehrere thermoelektrische Kanäle aufweist, die mit dem einen oder den mehreren piezoelektrischen Kanälen korrespondieren.

## Revendications

1. Sonde à ultrasons (110) comprenant :
une couche piézoélectrique (11) qui vibre et génère des ondes ultrasonores si un courant est fourni à la couche piézoélectrique (11) ;
un élément thermoélectrique (15) qui vient en contact avec une surface d'un circuit intégré à application spécifique (ASIC, 11a) et commande la température de la couche piézoélectrique (11) si un courant est fourni à l'élément thermoélectrique (15), la surface inférieure de l'élément thermoélectrique (15) étant une plaque de dissipation de chaleur et la surface supérieure de l'élément thermoélectrique (15) étant une plaque d'absorption de chaleur, et
l'ASIC (11a) étant disposé entre la couche piézoélectrique (11) et l'élément thermoélectrique (15), dans lequel l'ASIC (11a) établit un contact de surface avec la surface inférieure de la couche piézoélectrique (11) et est configuré pour commander un courant transmis à la couche piézoélectrique (11) ;
une électrode de signal partagée (16) disposée sur la surface inférieure de l'élément thermoélectrique (15) et configurée pour fournir un courant ; une électrode de masse partagée (17) disposée sur la surface supérieure de la couche piézoélectrique (11) et configurée pour décharger un courant circulant à travers l'ASIC (11a), l'élément thermoélectrique (15) et la couche piézoélectrique (11).

2. Sonde à ultrasons (110) selon la revendication 1, dans laquelle la couche piézoélectrique (11) et l'élément thermoélectrique (15) partagent une électrode de signal (16a, 16b) à travers laquelle un courant est fourni à la couche piézoélectrique (11) et à l'élément thermoélectrique (15), respectivement.

3. Sonde à ultrasons (110) selon la revendication 1, dans laquelle la couche piézoélectrique (11) et l'élément thermoélectrique (15) partagent une électrode de masse (17a, 17b).

4. Sonde à ultrasons (110) selon la revendication 1, dans laquelle, lorsque la couche piézoélectrique (11) comprend un ou plusieurs canaux piézoélectriques, l'élément thermoélectrique (15) comprend un ou plusieurs canaux thermoélectriques correspondant aux un ou plusieurs canaux piézoélectriques.
